# EUROPEAN PATENT APPLICATION

(11) **EP 4 851 133 A1**
(43) Date of publication of application: **09.12.2026**
(21) Application number: 25823550.6
(22) Date of filing: 10.03.2025
(51) Int. Cl.: C07C 69/734, A61K 8/37, A61K 8/49, A61K 31/36, A61K 31/216, A61P 17/00, A61P 35/00, A61Q 19/00, A61Q 19/08, C07D 317/60

(54) **BAKUCHIOL DERIVATIVE, PREPARATION METHOD THEREFOR, AND USE THEREOF**

(30) Priority: 13.09.2024 CN 202411291863
(71) Applicant: Shenzhen Hujia Technology Co., Ltd., Shenzhen, Guangdong 518066 (CN)
(72) Inventor: SHU, Peng, Shenzhen, Guangdong 518066 (CN); ZHONG, Jiangming, Shenzhen, Guangdong 518066 (CN); LIU, Zhao, Shenzhen, Guangdong 518066 (CN); LIANG, Ling, Shenzhen, Guangdong 518066 (CN); LIU, Qin, Shenzhen, Guangdong 518066 (CN)
(74) Representative: Huang, Liwei
(86) International application number: PCT/CN2025/081486
(87) International publication number: WO 2026/056233

(57) **Abstract**

Embodiments of this disclosure disclose bakeuchiol derivatives, and preparation methods and uses of bakeuchiol derivatives. The bakeuchiol derivatives include bakeuchiol derivatives with the structure of formula (I) or bakeuchiol derivatives with the structure of formula (II).

## Description

### CROSS-REFERENCE TO RELATED APPLICATIONS

This application claims the priority right of the Chinese patent application for the invention titled "bakeuchiol derivatives, and preparation methods and uses of bakeuchiol derivatives" and filed on September 13, 2024 under the application number 2024112918639, the entire contents of which are incorporated into this application as a whole.

### FIELD OF THE INVENTION

Embodiments of this disclosure relate to the field of biotechnology, and in particular, to bakeuchiol derivatives, and preparation methods and uses of bakeuchiol derivatives.

### BACKGROUND OF THE INVENTION

In recent years, drug development based on the synergistic mechanism of drug active molecules has gradually become one of the research hotspots in the field of biotechnology. This approach aims to achieve synergistic effects by acting on multiple targets or signaling pathways in a combined manner, thereby achieving more effective results compared to a single drug. At present, the drug development method based on the synergistic mechanism of drug active molecules is, for example, to connect two compounds through chemical bonds to construct new drug active molecules. The drugs obtained based on this drug development method are usually called synergistic drugs. To be specific, synergistic drugs are new molecules that connect two different drugs through covalent bonds, with the aim of achieving concentrated drug effects and enhancing synergistic interactions between drugs. For example, when combined with the β-lactase inhibitor Sulbactam, the antibiotic ampicillin can exert its antibacterial effect without being hydrolyzed by resistant bacteria, thereby effectively solving the problem of ampicillin resistance.

Bakeuchiol (BKU) is a monoterpene compound isolated from the leguminous plant psoralea. It can be used to treat skin dullness and pigment deposition after inflammation; can inhibit free radical induced lipid peroxidation; prevent UV damage to the skin and delay cell apoptosis; reduce the breakdown of collagen and promote the synthesis of collagen. In addition, bakeuchiol has a high degree of mildness, low irritation to the skin, and no photosensitivity.

However, there are often the following technical issue concerning bakeuchiol:

How to develop a kind of novel active molecules with lower cytotoxicity and multiple functions based on bakeuchiol has become an urgent problem to be solved.

The above information disclosed in this Background section is only for enhancement of understanding of the background of this inventive concept and therefore may contain information that does not form the prior art known to a person having ordinary skill in the art.

### SUMMARY OF THE INVENTION

The content of this disclosure is to briefly introduce concepts, which will be described in detail in the specific implementation section later. This disclosure is not intended to identify key or essential features of the claimed technical solution, nor is it intended to limit the scope of the claimed technical solution.

Some embodiments of this disclosure propose bakeuchiol derivatives, and preparation methods and uses of bakeuchiol derivatives, to address one or more of the technical issues mentioned in the background section above.

In the first aspect, some embodiments of this disclosure provide a bakeuchiol derivative, comprising: a bakeuchiol derivative with the structure of formula (I) or a bakeuchiol derivative with the structure of formula (II), wherein formula (I) is: 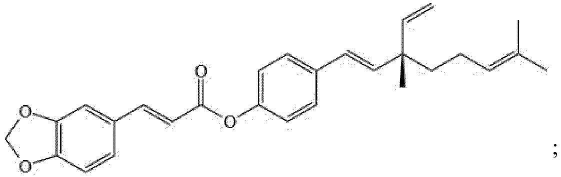 and formula (II) is:

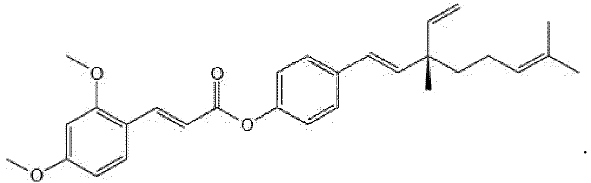

In the second aspect, some embodiments of this disclosure provide a method of preparing bakeuchiol derivatives, comprising: mixing 3- (benzo [d] [1,3] dioxolan-5-yl) acrylic acid, bakeuchiol, 1-ethyl-(3-dimethylaminopropyl) carbodiimide hydrochloride, 4-dimethylaminopyridine, triethylamine, and tetrahydrofuran, and stirring evenly at room temperature to obtain a mixture; performing a first vacuum concentration to the mixture to obtain a first concentrate; performing extraction on the first concentrate dissolved in water for a target number of times by ethyl acetate, and collecting an organic layer; performing a second vacuum concentration to the dried organic layer above to obtain a second concentrate; adding petroleum ether to the second concentrate and performing a third vacuum concentration to obtain a first solid; mixing a mixed solution with the first solid, then beating and filtering to obtain a filter cake, wherein the mixed solution comprises petroleum ether and ethyl acetate; vacuum drying the filter cake to obtain a bakeuchiol derivative, wherein the bakeuchiol derivative is a bakeuchiol derivative with the structure of formula (I) as described in the first aspect.

In the third aspect, some embodiments of this disclosure provide a method of preparing bakeuchiol derivatives, comprising: mixing 2,4-dimethoxycinnamic acid, bakeuchiol, 1-ethyl-(3-dimethylaminopropyl) carbodiimide hydrochloride, 4-dimethylaminopyridine, triethanolamine, and tetrahydrofuran, and stirring evenly at room temperature to obtain a mixture; performing a first vacuum concentration to the mixture to obtain a first concentrate; performing extraction on the first concentrate dissolved in water for a target number of times by ethyl acetate, and collecting an organic layer; performing a second vacuum concentration to the dried organic layer above to obtain a second concentrate; adding petroleum ether to the second concentrate and performing a third vacuum concentration to obtain a first solid; mixing a mixed solution with the first solid, then beating and filtering to obtain a filter cake, wherein the mixed solution comprises petroleum ether and ethyl acetate; vacuum drying the filter cake to obtain a bakeuchiol derivative, wherein the bakeuchiol derivative is a bakeuchiol derivative with the structure of formula (II) as described in the first aspect.

In the fourth aspect, some embodiments of this disclosure provide a skincare composition, comprising: a bakeuchiol derivative, wherein the bakeuchiol derivative is a bakeuchiol derivative with the structure of formula (I) or a bakeuchiol derivative with the structure of formula (II) as described in the first aspect; and a physiologically acceptable excipient or carrier.

Optionally, the bakeuchiol derivative is 0.01% to 5% by weight relative to the total weight of the skincare composition.

Optionally, the bakeuchiol derivative is 0.01% to 0.1% by weight relative to the total weight of the skincare composition.

Optionally, the bakeuchiol derivative is 0.01% to 0.05% by weight relative to the total weight of the skincare composition.

Optionally, the bakeuchiol derivative is 0.05% to 0.1% by weight relative to the total weight of the skincare composition.

Optionally, the bakeuchiol derivative is 0.1% to 0.2% by weight relative to the total weight of the skincare composition.

Optionally, the bakeuchiol derivative is 0.2% to 0.3% by weight relative to the total weight of the skincare composition.

Optionally, the bakeuchiol derivative is 0.3% to 0.4% by weight relative to the total weight of the skincare composition.

Optionally, the bakeuchiol derivative is 0.4% to 0.5% by weight relative to the total weight of the skincare composition.

Optionally, the bakeuchiol derivative is 0.5% to 0.6% by weight relative to the total weight of the skincare composition.

Optionally, the bakeuchiol derivative is 0.6% to 0.7% by weight relative to the total weight of the skincare composition.

Optionally, the bakeuchiol derivative is 0.7% to 0.8% by weight relative to the total weight of the skincare composition.

Optionally, the bakeuchiol derivative is 0.8% to 0.9% by weight relative to the total weight of the skincare composition.

Optionally, the bakeuchiol derivative is 0.9% to 1% by weight relative to the total weight of the skincare composition.

Optionally, the bakeuchiol derivative is 1% to 1.2% by weight relative to the total weight of the skincare composition.

Optionally, the bakeuchiol derivative is 1.2% to 1.4% by weight relative to the total weight of the skincare composition.

Optionally, the bakeuchiol derivative is 1.4% to 1.6% by weight relative to the total weight of the skincare composition.

Optionally, the bakeuchiol derivative is 1.6% to 1.8% by weight relative to the total weight of the skincare composition.

Optionally, the bakeuchiol derivative is 1.8% to 2% by weight relative to the total weight of the skincare composition.

Optionally, the bakeuchiol derivative is 2% to 2.5% by weight relative to the total weight of the skincare composition.

Optionally, the bakeuchiol derivative is 2.5% to 3% by weight relative to the total weight of the skincare composition.

Optionally, the bakeuchiol derivative is 3% to 3.5% by weight relative to the total weight of the skincare composition.

Optionally, the bakeuchiol derivative is 3.5% to 4% by weight relative to the total weight of the skincare composition.

Optionally, the bakeuchiol derivative is 4% to 5% by weight relative to the total weight of the skincare composition.

Optionally, the skincare composition further includes one or more of: organic solvents, oils, waxes, pigments, fillers, dyes, surfactants, emulsifiers, cosmetic surfactants, organic or inorganic photoprotective agents, polymers, thickeners, preservatives, fragrances, fungicides, actives, retinol, retinol derivatives, flavorings, antioxidants, and rheology modifiers.

Optionally, the form of the skincare composition includes one or more of: solutions, water agents, lotion, creams, cleaning agents, ointments, perfumes, gel, pastes, powders, sprays, inhalants, and patches.

Optionally, the water agents may include one or more of: skincare water, toner and essence.

Optionally, the cleaning agents may include one or more of: face cleaning foam, face cleaning water and face cleaning liquid.

Optionally, the actives include one or more of: small molecules, peptides, proteins, carbohydrates, monosaccharides, oligosaccharides, polysaccharides, nucleoproteins, mucins, synthetic peptides, small molecules linked to proteins, glycoproteins, steroids, nucleic acids, deoxyribonucleic acids, ribonucleic acids, nucleosides, nucleotides, oligonucleotides, antisense oligonucleotides, lipids, hormones, vitamin A, vitamin A derivative, cells.

In the fifth aspect, some embodiments of this disclosure provide a use of bakeuchiol derivatives, which comprises any of the following uses of bakeuchiol derivatives with the structure of formula (I) or with the structure of formula (II) as described in the first aspect: anti-aging; inhibiting cell apoptosis; regulating the cell cycle; inhibiting deoxyribonucleic acid damage; inhibiting tumors.

In the sixth aspect, some embodiments of this disclosure provide any of the following uses of the skincare composition as described in the fourth aspect: anti-aging; inhibiting cell apoptosis; regulating the cell cycle; inhibit deoxyribonucleic acid damage; inhibiting tumors.

### BRIEF DESCRIPTION OF THE DRAWINGS

The above and other features, advantages, and aspects of the embodiments of this disclosure will become more apparent in conjunction with the accompanying drawings and with reference to the following specific implementations. Throughout the drawings, the same or similar reference signs indicate the same or similar elements. It should be understood that the drawings are schematic, and the elements are not necessarily drawn to scale.
Figure 1 is a schematic diagram of the synthesis process of bakeuchiol derivatives with the structure of formula (I) in some embodiments of this disclosure;
Figure 2 is a mass spectrum of bakeuchiol derivatives with the structure of formula (I) in some embodiments of this disclosure;
Figure 3 is a schematic diagram of the synthesis process of bakeuchiol derivatives with the structure of formula (II) in some embodiments of this disclosure;
Figure 4 is a mass spectrum of bakeuchiol derivatives with the structure of formula (II) in some embodiments of this disclosure;
Figure 5 is a comparison chart of cell survival rates corresponding to Experiment 2 in some embodiments of this disclosure;
Figure 6 is a comparison chart of relative expression levels of Caspase3 corresponding to Experiment 3 in some embodiments of this disclosure;
Figure 7 is a comparison chart of relative expression levels of Caspase3 corresponding to Experiment 3 in some embodiments of this disclosure;
Figure 8 is a comparison chart of relative expression levels of WIP1 corresponding to Experiment 4 in some embodiments of this disclosure;
Figure 9 is a comparison chart of relative expression levels of WIP1 corresponding to Experiment 4 in some embodiments of this disclosure;
Figure 10 is a comparison chart of relative expression levels of Caspase3 corresponding to Experiment 5 in some embodiments of this disclosure;
Figure 11 is a comparison chart of relative expression levels of WIP1 corresponding to Experiment 5 in some embodiments of this disclosure.

### DETAILED DESCRIPTION OF THE INVENTION

Hereinafter, the embodiments of this disclosure will be described in more detail with reference to the accompanying drawings. Although certain embodiments of this disclosure are shown in the drawings, it should be understood that this disclosure may be implemented in various forms, and shall not be construed as being limited to the embodiments set forth herein. On the contrary, these embodiments are provided for a more thorough and complete understanding of this disclosure. It should be understood that the drawings and embodiments of this disclosure are used only for illustrative purposes, not to limit the protection scope of this disclosure.

Besides, it should be noted that, for ease of description, only the portions related to the relevant invention are shown in the drawings. In the case of no conflict, the embodiments in this disclosure and the features in the embodiments may be combined with each other.

It should be noted that such adjuncts as 'one' and 'more' mentioned in this disclosure are illustrative, not restrictive, and those skilled in the art should understand that, unless the context clearly indicates otherwise, they should be understood as 'at least one'.

It should be noted that the term 'about' mentioned in this disclosure refers to ± 5% of the specified value when used to limit a numerical value.

It should be noted that the term '%' mentioned in this disclosure, unless otherwise specified, refers to mass%.

It should be noted that the term "room temperature" mentioned in this disclosure refers to the ambient temperature, which has not undergone any special heating or cooling treatment.

### Explanation of Terminology

The term 'bakeuchiol' is a monoterpene compound isolated from the leguminous plant psoralea, with a chemical formula of C₁₈H₂₄O, the structural formula is as follows:

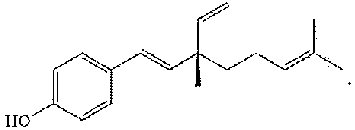

The term 'ferulic acid' is a hydroxyphenylacrylic acid, which is a rich phenolic phytochemical component present in plant cell walls, with the following structural formula:

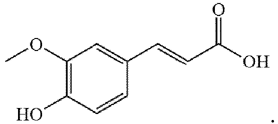

The chemical formula for the term '3-(benzo [d] [1,3] dioxolan-5-yl) acrylic acid' is C₁₀H₈O₄, with the following structural formula:

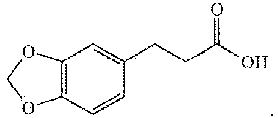

The chemical formula for the term '2,4-dimethoxycinnamic acid' is C₁₁H₁₂O₄, with the following structural formula:

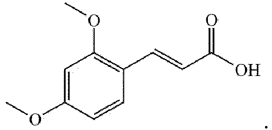

The term 'compound of formula (III)' refers to a graft of ferulic acid and bakeuchiol, with the following structural formula:

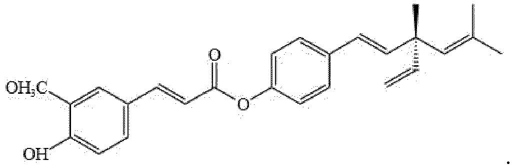

The chemical formula for the term "1-Ethyl-(3-dimethylaminopropyl) carbodiimide hydrochloride" is C₈H₁8CₗN₃, abbreviated as EDCI.

The term "4-dimethylaminopyridine" is a pyridine derivative, which is a colorless to pale yellow crystal with the chemical formula C₇H₁₀N₂, abbreviated as DMAP.

The term "triethylamine" is an amine organic compound that is a colorless to pale yellow transparent liquid with a pungent fishy odor, extremely bitter taste, and slightly smoky in the air; its chemical formula is C₆H₁₅N, abbreviated as ET₃N.

The term 'tetrahydrofuran', also known as oxacyclopentane, is a heterocyclic organic compound that is a colorless and transparent liquid with the chemical formula C₄H₈O, abbreviated as THF.

The term 'ethyl acetate' is a compound formed by replacing the hydroxyl group in acetic acid with an ethoxy group, with a chemical formula of C₄H₈O₂.

The term 'petroleum ether' is a light petroleum product mainly composed of pentane and hexane, being is a colorless, highly flammable, transparent liquid with a kerosene odor.

The term 'triethanolamine' is a light yellow liquid with the chemical formula C₆H₁₅NO₃.

The term 'ampicillin', also known as ampicillin, is an organic compound with the chemical formula C₁₆H₁₉N₃O₄S.

The term 'shubatan' refers to irreversible competitive β-lactase inhibitors, having an inhibitory effect on all the β-lactase produced by Gram positive bacteria and negative bacteria other than pseudomonas aeruginosa, with the chemical formula C₁₄H₂₁NO₇S.

The term 'anhydrous sodium sulfate' is an ionic sulfate salt that appears as white or light yellow granular crystals or powders, with a chemical formula of Na₂O₄S.

The term 'dimethyl sulfoxide' is also known as DMSO, which is a colorless polar non-proton solvent with the chemical formula (CH₃)₂SO.

The technical problem to be solved in this disclosure is how to develop a novel active molecule with lower cytotoxicity and multiple functions based on bakeuchiol.

In order to solve the problem above, some embodiments of this disclosure propose bakeuchiol derivatives, namely bakeuchiol derivatives with the structure of formula (I) or with the structure of formula (II), which achieve the grafting of bakeuchiol and ferulic acid, resulting in bakeuchiol derivatives with low toxicity and various effects such as inhibiting cell apoptosis and regulating cell cycle.

Below, this disclosure will be explained in detail with reference to the accompanying drawings and in conjunction with embodiments.

### Description of Preparation Methods

Bakeuchiol derivative preparation method A comprises:
Step 1: Mixing 3-(benzo [d] [1,3] dioxolan-5-yl) acrylic acid, bakeuchiol, 1-ethyl-(3-dimethylaminopropyl) carbodiimide hydrochloride, 4-dimethylaminopyridine, triethylamine, and tetrahydrofuran, and stirring evenly at room temperature to obtain a mixture.

In practice, the mixture can be obtained by adding 3-(benzo [d] [1,3] dioxolan-5-yl) acrylic acid 4.5 g (22.6 mmol, 1.5 equiv), bakeuchiol 4.5 g (15 mmol, 1 equiv), 1-Ethyl-(3-dimethylaminopropyl) carbodiimide hydrochloride 5.76 g (30 mmol, 2 equiv), 4-dimethylaminopyridine 180 mg (1.5 mmol, 0.1 equiv), triethylamine 4.8 mL (37.6mmol, 2.5 equiv) and tetrahydrofuran 80 mL, to a reaction kettle of 200 mL, stirring at room temperature for 12 hours.

Step 2: Performing a first vacuum concentration to the mixture to obtain a first concentrate.

Wherein, a vacuum concentration is performed to the mixture at a temperature of 40 ± 5 °C to obtain the first concentrate.

Step 3: Performing extraction on the first concentrate dissolved in water for a target number of times by ethyl acetate, and collecting an organic layer.

In practice, adding 200 mL of water to the first concentrate to dissolve the first concentrate in water. Then, performing extraction on the first concentrate dissolved in water for a target number of times by ethyl acetate (200 mL × 3). Wherein, the target number is ≥ 1. Finally, merging the organic layer.

Step 4: Performing a second vacuum concentration to the dried organic layer above to obtain a second concentrate.

In practice, first, adding 10 g of anhydrous sodium sulfate to the organic layer, drying and filtering the organic layer to obtain a dried organic layer. Then, under temperature conditions of 40 ± 5 °C, performing a vacuum concentration to the dried organic layer above to obtain a second concentrate (oily crude product).

Step 5: Adding petroleum ether to the second concentrate and performing a third vacuum concentration to obtain a first solid.

In practice, adding 60 mL of petroleum ether to the second concentrate, and performing a vacuum concentration under a temperature of 40 ± 5 °C to obtain the first solid (yellow solid).

Step 6: Mixing a mixed solution with the first solid, then beating and filtering to obtain a filter cake.

Wherein, the mixed solution comprises petroleum ether and ethyl acetate, the volume ratio of petroleum ether to ethyl acetate being 50:1.

In practice, 200 mL of mixed solution comprised of petroleum ether and ethyl acetate is mixed and beaten with the first solid for 30 minutes, and filtered through a Buchner funnel to obtain a filter cake.

Step 7: Vacuum drying the filter cake to obtain a bakeuchiol derivative.

Wherein, the bakeuchiol derivative is a bakeuchiol derivative with the structure of formula (I).

In practice, the filter cake wi vacuum dried at a temperature of 40 ± 5 °C to obtain a bakeuchiol derivative (4 g, yield: 62%).

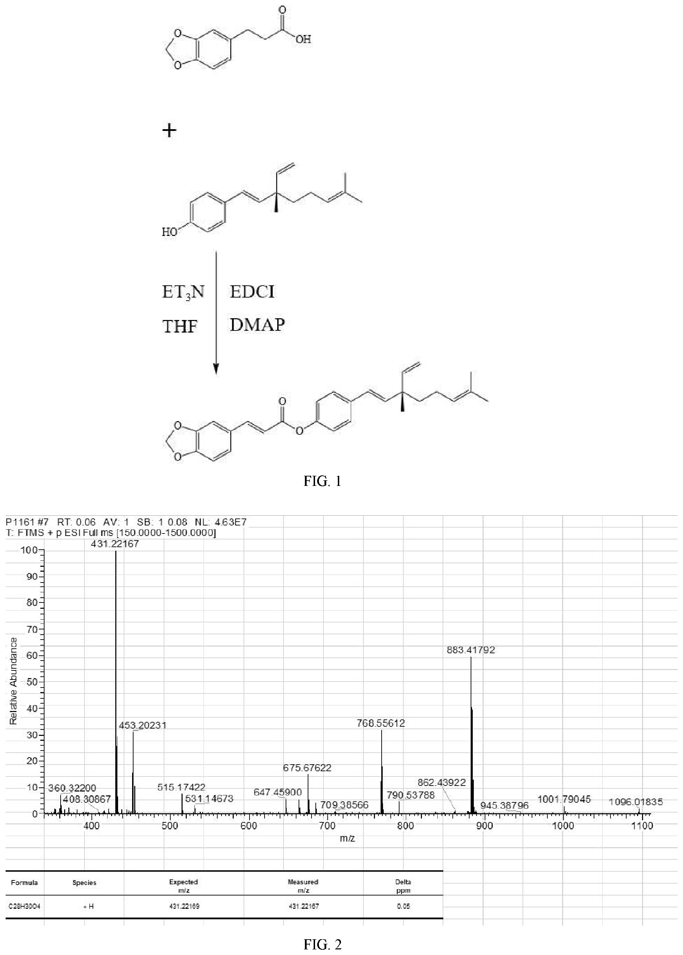

To be specific, the schematic diagram of the synthesis process of bakeuchiol derivatives with the structure of formula (I) is shown in Figure 1. The mass spectrum of bakeuchiol derivatives with the structure of formula (II) is shown in Figure 2.

To be specific, the NMR spectral attribution result of bakeuchiol derivatives with the structure of formula (I) is as follows:
¹H NMR (400 MHz, CDCl3): δ = 7.77 (d, J = 15.9 Hz, 1H), 7.39 (d, J = 8.6 Hz, 2H), 7.15-7.03 (m, 4H), 6.84 (d, J = 7.9 Hz, 1H), 6.44 (d, J = 15.8 Hz, 1H), 6.38-6.13 (m, 2H), 6.03 (s, 2H), 5.89 (dd, J = 17.5, 10.7 Hz, 1H), 5.16-4.99 (m, 3H), 1.97 (q, J = 7.5 Hz, 2H), 1.69 (s, 3H), 1.60 (s, 3H), 1.56-1.49 (m, 2H), 1.22 (s, 3H).
¹³C NMR (101 MHz, CDCl3): δ = 165.59, 149.95, 149.71 ,148.42, 146.20, 145.65, 138.07, 135.56, 131.33, 128.60, 126.94, 126.32, 124.86, 124.68, 121.59, 115.09, 112.08, 108.60, 106.56, 101.62, 42.62, 41.19, 25.66, 23.27, 23.19, 17.62.

Wherein, the nuclear magnetic data is obtained through a nuclear magnetic resonance spectrometer (Model: AVANCE III HD 400MHz; Manufacturer: Switzerland Bruker).

Bakeuchiol derivative preparation method B comprises:
Step 1: Mixing 2,4-dimethoxycinnamic acid, psoralen, 1-ethyl - (3-dimethylaminopropyl) carbodiimide hydrochloride, 4-dimethylaminopyridine, triethanolamine, and tetrahydrofuran, and stirring evenly at room temperature to obtain a mixture.

In practice, the mixture can be obtained by adding 2,4-dimethoxycinnamic acid 468 mg (2.25 mmol, 1.5 equiv), bakeuchiol 384 mg (1.5mmol, 1 equiv), 1-Ethyl-(3-dimethylaminopropyl) carbodiimide hydrochloride 465 mg (3 mmol, 2 equvi), 4-dimethylaminopyridine 18 mg (0.15 mmol, 0.1equiv), triethanolamine 0.48 mL (3.75 mmol, 2.5equiv) and tetrahydrofuran 1 mL, to a reaction kettle of 200 mL, stirring at room temperature for 12 hours.

Step 2: Performing a first vacuum concentration to the mixture to obtain a first concentrate.

Wherein, a first vacuum concentration is performed to the mixture at a temperature of 40 ± 5 °C to obtain the first concentrate.

Step 3: Performing extraction on the first concentrate dissolved in water for a target number of times by ethyl acetate, and collecting an organic layer.

In practice, adding 200 mL of water to the first concentrate to dissolve the first concentrate in water. Then, performing extraction on the first concentrate dissolved in water for a target number of times by ethyl acetate (200 mL × 3). Wherein, the target number is ≥ 1. Finally, merging the organic layer.

Step 4: Performing a second vacuum concentration to the dried organic layer above to obtain a second concentrate.

In practice, first, adding 10 g of anhydrous sodium sulfate to the organic layer, drying and filtering the layer to obtain a dried organic layer. Then, under temperature conditions of 40 ± 5 °C, performing a vacuum concentration to the dried organic layer above to obtain a second concentrate (oily crude product).

Step 5: Adding petroleum ether to the second concentrate and performing a third vacuum concentration to obtain a first solid.

In practice, adding 10 mL of petroleum ether to the second concentrate, and performing a vacuum concentration under a temperature of 40 ± 5 °C to obtain the first solid (yellow solid).

Step 6: Mixing a mixed solution with the first solid, then beating and filtering to obtain a filter cake.

Wherein, the mixed solution comprises petroleum ether and ethyl acetate, the volume ratio of petroleum ether to ethyl acetate being 50:1.

In practice, 200 mL of mixed solution comprised of petroleum ether and ethyl acetate is mixed and beaten with the first solid for 30 minutes, and filtered through a Buchner funnel to obtain a filter cake.

Step 7: Vacuum drying the filter cake to obtain a bakeuchiol derivative.

Wherein, the bakeuchiol derivative is a bakeuchiol derivative with the structure of formula (II).

In practice, the filter cake wi vacuum dried at a temperature of 40 ± 5 °C to obtain a bakeuchiol derivative (442 g, yield: 66%).

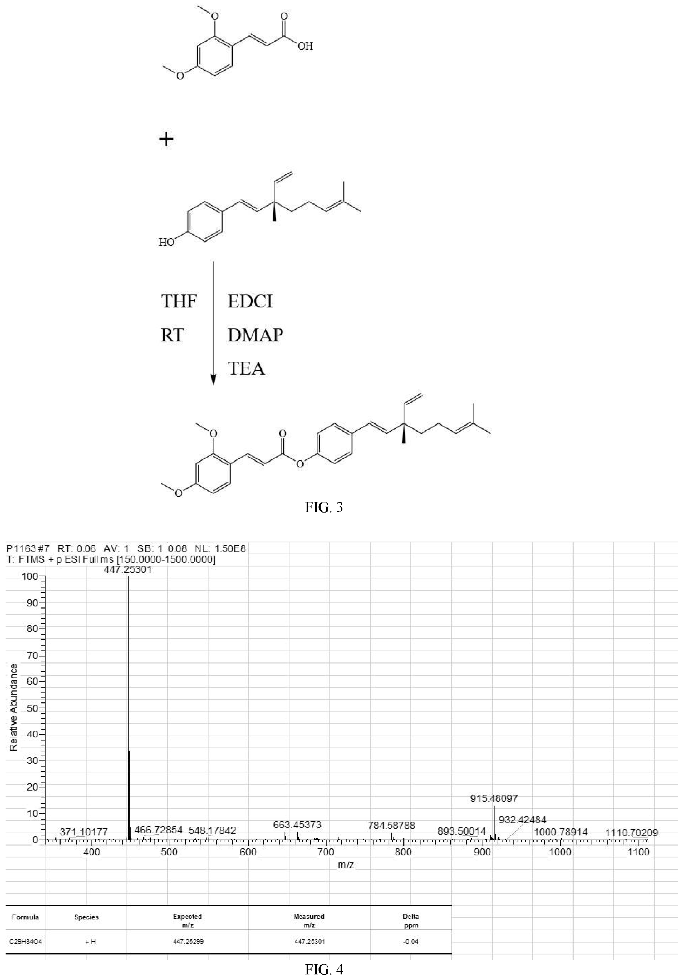

To be specific, the schematic diagram of the synthesis process of bakeuchiol derivatives with the structure of formula (II) is shown in Figure 3. Wherein, the 'RT' in Figure 3 means Room Temperature. The mass spectrum of bakeuchiol derivatives with the structure of formula (II) is shown in Figure 4.

To be specific, the NMR spectral attribution result of bakeuchiol derivatives with the structure of formula (II) is as follows:
¹H NMR (400 MHz, CDCl3): δ = 8.09 (d, J = 16.1 Hz, 1H), 7.48 (d, J = 8.6 Hz, 1H), 7.39 (d, J = 8.7 Hz, 2H), 7.11 (d, J = 8.6 Hz, 2H), 6.63 (d, J = 16.0 Hz, 1H), 6.52 (dd, J = 8.6, 2.4 Hz, 1H), 6.46 (d, J = 2.3 Hz, 1H), 6.38-6.13 (m, 2H), 5.90 (dd, J = 17.4, 10.7 Hz, 1H), 5.19-5.01 (m, 3H), 3.87 (s, 3H), 3.83 (s, 3H), 1.98 (q, J = 7.6 Hz, 2H), 1.69 (s, 3H), 1.60 (s, 3H), 1.57-1.46 (m, 2H), 1.22 (s, 3H).

¹³C NMR (101 MHz, CDCl3): δ = 166.19, 162.97, 159.99, 149.87, 145.56, 141.85, 137.76, 135.22, 131.13, 130.75, 126.77, 126.30, 124.62, 121.61, 116.20, 114.80, 111.96, 105.26, 98.26, 55.30, 55.29, 42.50, 41.10, 25.56, 23.16, 23.10, 17.51.

Wherein, the nuclear magnetic data is obtained through a nuclear magnetic resonance spectrometer (Model: AVANCE III HD 400MHz; Manufacturer: Switzerland Bruker).

### Description of Experimental Methods

### Experiment 1 (Cytotoxicity Experiment)

Wherein, Experiment 1 used a control, a sample 1, a sample 2, a sample 3, and a sample 4. The specific description of these samples is as follows:
Control: A DMEM culture medium containing 10% FBS and 1% penicillin/streptomycin.

Sample 1: First, mixing the bakeuchiol into DMSO to form a mother liquor. Then, diluting the mother liquor to the required concentration for the experiment by means of the DMEM culture medium containing 10% FBS and 1% penicillin/streptomycin.

Sample 2: First, mixing the compound of formula (III) into DMSO to form a mother liquor. Then, diluting the mother liquor to the required concentration for the experiment by means of the DMEM culture medium containing 10% FBS and 1% penicillin/streptomycin.

Sample 3: First, mixing the bakeuchiol derivative with the structure of formula (I) into DMSO to form a mother liquor. Then, diluting the mother liquor to the required concentration for the experiment by means of the DMEM culture medium containing 10% FBS and 1% penicillin/streptomycin.

Sample 4: First, mixing the bakeuchiol derivative with the structure of formula (II) into DMSO to form a mother liquor. Then, diluting the mother liquor to the required concentration for the experiment by means of the DMEM culture medium containing 10% FBS and 1% penicillin/streptomycin.

### Description of Experimental Process

Step 1: Inoculation of HDF (Human Dermal Fibroblasts) cells.

Wherein, inoculating to a 96-well plate at a density of 2 × 10⁴ cells/well, or to be specific, adding to each well 100 µL of DMEM culture medium containing 10% FBS and 1% penicillin/streptomycin (complete culture medium), and cultivating for 24 hours in a culture box (culture temperature: 37 °C; culture environment: 5% CO₂).

Step 2: Proceeding with drug administration when the cell spreading rate in the 96-well plate reaches 40%~50%.

Wherein, adding 100 µL of sample to each well. Setting up 6 sub-wells per group and placing them in a culture box (culture temperature: 37 °C; culture environment: 5%) after drug administration is completed CO₂), cultivating for 24 hours therein.

Step 3: Discarding the supernatant after 24 hours of cell incubation and adding 100 µL of CCK8 working fluid (prepared through the DMEM culture medium), incubating at 37 °C in the dark for 2 hours; after the incubation is complete, reading the OD value (relative vitality) at 450 nm and 650 nm, respectively.

Step 4: Determining a proliferation rate based on the blank control group.

Wherein, the proliferation rate = (relative vitality of experimental group - relative vitality of blank control group)/relative vitality of blank control group.

Explanation of Experimental Results

Wherein, the experimental results corresponding to Experiment 1 are shown in Table 1:

**Table 1**

| Sample Names | Sample Concentration | | | |
|---|---|---|---|---|
| | 0 µM | 12.5 µM | 25 µM | 50 µM |
| Control | 100% | / | / | / |
| Sample 1 | / | 97.50% | 96.83% | 82.99% |
| Sample 2 | / | 98.07% | 97.87% | 72.71% |
| Sample 3 | / | 104.54% | 98.78% | 93.88% |
| Sample 4 | / | 103.37% | 99.16% | 89.00% |

Analysis reveals that, firstly, in the concentration range of 12.5 µM ~ 50 µM, sample 1, sample 3, and sample 4 did not produce cytotoxicity to the HDF cells. Secondly, the cell proliferation rate of sample 2, which has a concentration of 50 µM, is 72.71%, indicating cytotoxicity.

### Experiment 2 (Phototoxicity Experiment)

Wherein, Experiment 2 used a control, a UVA control (UVA), a sample 1, a sample 2, a sample 3, and a sample 4. The specific description of these sample is as follows:

Control: A DMEM culture medium containing 10% FBS and 1% penicillin/streptomycin.

UVA control (UVA): A DMEM culture medium containing 10% FBS and 1% penicillin/streptomycin.

Sample 1: First, mixing the bakeuchiol into DMSO to form a mother liquor. Then, diluting the mother liquor to the required concentration for the experiment by means of the DMEM culture medium containing 10% FBS and 1% penicillin/streptomycin.

Sample 2: First, mixing the compound of formula (III) into DMSO to form a mother liquor. Then, diluting the mother liquor to the required concentration for the experiment by means of the DMEM culture medium containing 10% FBS and 1% penicillin/streptomycin.

Sample 3: First, mixing the bakeuchiol derivative with the structure of formula (I) into DMSO to form a mother liquor. Then, diluting the mother liquor to the required concentration for the experiment by means of the DMEM culture medium containing 10% FBS and 1% penicillin/streptomycin.

Sample 4: First, mixing the bakeuchiol derivative with the structure of formula (II) into DMSO to form a mother liquor. Then, diluting the mother liquor to the required concentration for the experiment by means of the DMEM culture medium containing 10% FBS and 1% penicillin/streptomycin.

### Description of Experimental Process

Step 1: Inoculation of HDF (Human Dermal Fibroblasts) cells.

Wherein, inoculating to a 96-well plate at a density of 2 × 10⁴ cells/well, or to be specific, adding to each well 100 µL of DMEM culture medium containing 10% FBS and 1% penicillin/streptomycin (complete culture medium), and cultivating for 24 hours in a culture box (culture temperature: 37 °C; culture environment: 5% CO₂).

Step 2: Proceeding with UVA exposure and drug administration when the cell spreading rate in the 96-well plate reaches 40%~50%.

Wherein, adding 100 µL of PBS to each well and carrying out 7 J of UVA irradiation, after which, 100 µL of sample to each well. Setting up 6 sub-wells per group and placing them in a culture box (culture temperature: 37 °C; culture environment: 5%) after drug administration is completed CO₂), cultivating for 24 hours therein.

Step 3: Discarding the supernatant after 24 hours of cell incubation and adding 100 µL of CCK8 working fluid (prepared through the DMEM culture medium), incubating at 37 °C in the dark for 2 hours; after the incubation is complete, reading the OD value (relative vitality) at 450 nm and 650 nm, respectively.

Step 4: Determining a proliferation rate based on the blank control group.

Wherein, the proliferation rate = (relative vitality of experimental group - relative vitality of blank control group)/relative vitality of blank control group.

### Explanation of Experimental Results

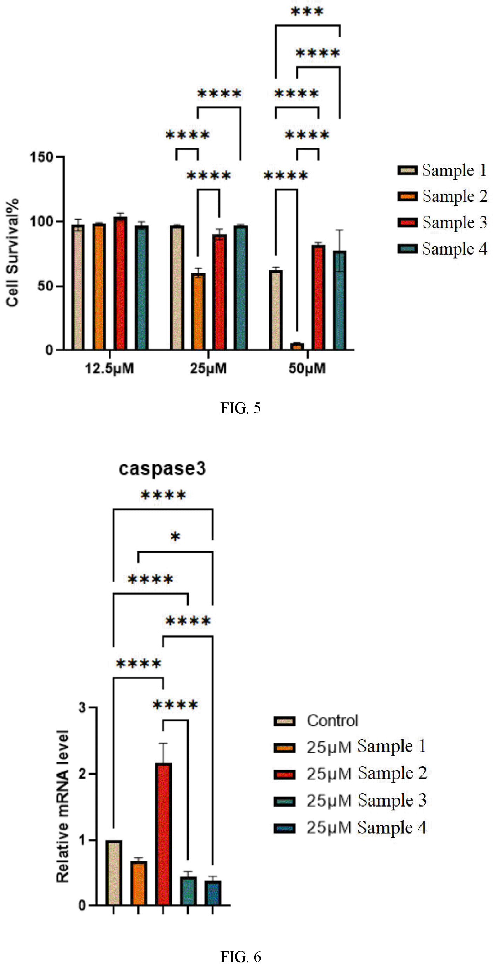

Wherein, the experimental results corresponding to Experiment 2 are shown in Table 2 and Figure 5:

**Table 2**

| Sample Names | Sample Concentration | | | |
|---|---|---|---|---|
| | 0 µM | 12.5 µM | 25 µM | 50 µM |
| Control | 100% | / | / | / |
| UVA control (UVA) | 82.13% | / | / | / |
| Sample 1 | / | 97.50% | 96.83% | 62.95% |
| Sample 2 | / | 98.78% | 60.45% | 5.57% |
| Sample 3 | / | 103.91% | 90.25% | 82.03% |
| Sample 4 | / | 97.12% | 96.83% | 77.58% |

Analysis reveals that, firstly, after UVA irradiation, the cell survival rate decreased. In addition, with the increase of concentration, the cytotoxicity of sample 1 gradually increased, and at the concentration of 50 µM, the cell survival rate was only 62.95%. Next, sample 2 had a cell survival rate of only 60.45% at the concentration of 25 µM, and a cell survival rate of only 5.57% at the concentration of 50 µM. Meanwhile, samples 3 and 4 at the concentration of 50 µM had the cell survival rates of 82.03% and 77.58% respectively. Wherein, samples 3 and 4 showed higher safety compared to samples 1 and 2.

### Experiment 3 (Caspase 3 Inhibition (Cell) Experiment)

Wherein, Caspase3 is a protease that plays a crucial role in the process of cell apoptosis. It is one of the executors of cell apoptosis, which triggers cell death by cleaving specific intracellular proteins. Therefore, the expression level of Caspase3 is often used as an indicator of cell apoptosis.

Wherein, Experiment 3 used a control, a sample 1, a sample 2, a sample 3, and a sample 4. The specific description of these samples is as follows:
Control: A DMEM culture medium containing 10% FBS and 1% penicillin/streptomycin.

Sample 1: First, mixing the bakeuchiol into DMSO to form a mother liquor. Then, diluting the mother liquor to the required concentration for the experiment by means of the DMEM culture medium containing 10% FBS and 1% penicillin/streptomycin.

Sample 2: First, mixing the compound of formula (III) into DMSO to form a mother liquor. Then, diluting the mother liquor to the required concentration for the experiment by means of the DMEM culture medium containing 10% FBS and 1% penicillin/streptomycin.

Sample 3: First, mixing the bakeuchiol derivative with the structure of formula (I) into DMSO to form a mother liquor. Then, diluting the mother liquor to the required concentration for the experiment by means of the DMEM culture medium containing 10% FBS and 1% penicillin/streptomycin.

Sample 4: First, mixing the bakeuchiol derivative with the structure of formula (II) into DMSO to form a mother liquor. Then, diluting the mother liquor to the required concentration for the experiment by means of the DMEM culture medium containing 10% FBS and 1% penicillin/streptomycin.

### Description of Experimental Process

Step 1: Inoculation of HDF (Human Dermal Fibroblasts) cells.

Wherein, inoculating to a 6-well plate at a density of 8 × 10⁵ cells/well, or to be specific, adding to each well 2000 µL of DMEM culture medium containing 10% FBS and 1% penicillin/streptomycin (complete culture medium), and cultivating for 24 hours in a culture box (culture temperature: 37 °C; culture environment: 5% CO₂).

Step 2: Grouping and administering drugs when the cell spreading rate in the 6-well plate reaches 70%~80%.

Wherein, adding complete culture media containing different concentrations of drugs, with 3 sub-wells set up in each group, and placed in a culture box (culture temperature: 37 °C; culture environment: 5% CO₂) after drug administration was completed, cultivating for 24 hours therein.

Step 3: Extracting RNA.

Wherein, (1), pour out the culture medium and use PBS to rinse once. To be specific, firstly, add 1 mL of TansZol^{TM} Up reagent to every 10 cm² of growing cultured cells, place it horizontally for a moment, distribute lysate evenly on the cell surface for cell lysis. Then, use a pipette to blow the cells and make them fall off (wherein, for firmly adhered cultured cells, use a cell scraper to peel them off). Next, transfer the cell lysate to a centrifuge tube and add 0.2 mL of RNA extraction agent (total RNA extraction reagent) and repeatedly blow and suck with a pipette until there is no obvious precipitate in the lysate, then vortex oscillate at room temperature for 5 minutes. (2) Centrifuge the sample between 2 °C and 8 °C for 15 minutes, at which point the sample is divided into three layers: the upper layer (colorless aqueous phase), the middle layer, and the lower layer (pink organic phase). Wherein, RNA is located in the aqueous phase, the volume of the aqueous phase is 50%~60% of the TansZol Up reagent (to avoid inhalation into the middle layer that results in DNA Pollution, a portion of aqueous phase may be retained appropriately). (3) Transfer the colorless aqueous phase to a new centrifuge tube and add an equal volume of anhydrous ethanol (precipitation may occur at this time), and gently mix them well. (4) Add the obtained solution and precipitate into a centrifuge column, centrifuge at room temperature for 30 seconds, and discard the effluent. (5) Add 500 µL of CB9, centrifuge at room temperature for 30 seconds, discard the effluent, and repeat the process once. (6) Add 500 µL of WB9 (before use, check if anhydrous ethanol has been added), centrifuge at room temperature for 30 seconds, discard the effluent, and repeat the process once. (7) Centrifuge at room temperature for 2 minutes to completely remove residual ethanol. (8) Place the centrifuge column in a RNase-freeTube, and add 50-200 µL of Nase-freeWater, let it rest at room temperature for 1 minute in the center of the centrifuge column. (9) Centrifuge at room temperature for 1 minute, wash off the RNA. (10) Use a micro spectrophotometer to conduct concentration measurement of the RNA and store the RNA at -80 °C.

Step 4. Calculating, by 2 ug, the corresponding RNA volume of the RNA sample that has measured concentration, and add the corresponding volume of Rnase free water and reverse transcriptase mix.

Wherein, the total volume of the system is 20 µL. To be specific, the RT-PCR reaction system and the RTPCR reaction program are shown in Table 3:

**Table 3**

| RT-PCR Reaction System | |
|---|---|
| Names | Volumes |
| RNA | 2 ug |
| Rnase Free Water | Up to 20 µL |
| Reverse Transcriptase Mix (5X Evo M-MLV RT Master Mix) | 4 µL |

| RT-PCR Reaction Program | |
|---|---|
| Temperatures | Times |
| 37°C | 40 min |
| 85°C | 5 s |
| 12°C | ∞ |

After the reverse transcription program is completed, dilute 20 µL of the reverse transcription sample to 400 µL, wherein, the final concentration of the cDNA sample is 5 ng/µL (wherein, 3 sub-wells were made for each gene in each sample for testing, and after adding samples, they were put on the machine for QPCR reaction program). To be specific, the QPCR reaction system and the QPCR reaction program are shown in Table 4:

**Table 4**

| QPCR Reaction System | |
|---|---|
| Names | Volumes |
| cDNA Templet | 4 µL |
| 2X QPCR SYBR Green Master Mix | 5 µL |
| ddH₂O | 0.4 µL |
| 10uM Caspase3 Primers (Forward+Reverse) | 0.6 µL |

| QPCR Reaction Program | |
|---|---|
| Temperatures | Times |
| 95°C | 5 min |
| 95°C | 10 s |
| 60°C | 20 s |
| 72°C(scan) | 30 s |
| 40X | |
| Melt (scan) | 15 s |

Wherein, 2-△△Ct is used for analyzing related genes' mRNA expression level, data between groups uses One-way ANOVA statistical analysis, P<0.05 indicates that the difference is significant and has statistical significance.

### Explanation of Experimental Results

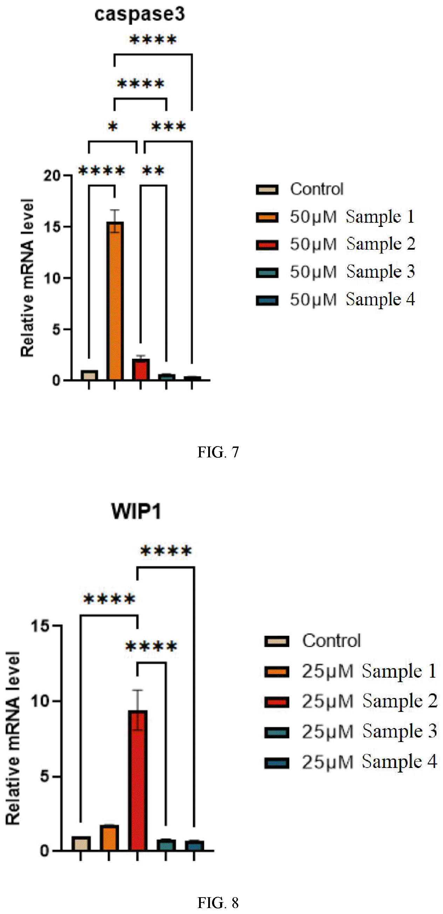

Wherein, the experimental results corresponding to Experiment 3 are shown in Table 5, Figure 6, and Figure 7:

**Table 5**

| Sample Names | Caspase3 Relative Expression Level | |
|---|---|---|
| Sample Concentration | 25 µM | 50 µM |
| Control | 1.000 | 1.000 |
| Sample 1 | 0.678 | 15.590 |
| Sample 2 | 2.171 | 2.143 |
| Sample 3 | 0.411 | 0.639 |
| Sample 4 | 0.384 | 0.418 |

Analysis reveals that, firstly, sample 1 had the effect of inhibiting the Caspase3 expression when at the concentration of 25 µM, and enhanced the Caspase3 expression when at the concentration of 50 µM, increasing by about 2200% compared to the control. Secondly, sample 2 improved the Caspase3 expression at both the concentration of 25 µM and 50 µM, increasing by about 100% compared to the control. Next, samples 3 and 4 both have the effect of inhibiting the Caspase3 expression when at the concentration of 25 µM and 50 µM, decreasing by about 50% compared to the control. In summary, samples 3 and 4, compared to sample 2, have completely different biological activities.

### Experiment 4 (WIP1 Inhibition (Cell) Experiment)

Wherein, WIP1 (Wild-type p53-induced phosphatase 1) is a phosphatase, belonging to Ser/Thr protein phosphatase family. It consists of p53 gene-induced expression and plays an important role in cell cycle regulation, DNA damage response and other processes. WIP1 expression level can be used as indicators for DNA damage response, cell cycle regulation, and tumor occurrence and development process.

Wherein, Experiment 4 used a control, a sample 1, a sample 2, a sample 3, and a sample 4. The specific description of these samples is as follows:
Control: A DMEM culture medium containing 10% FBS and 1% penicillin/streptomycin.

Sample 1: First, mixing the bakeuchiol into DMSO to form a mother liquor. Then, diluting the mother liquor to the required concentration for the experiment by means of the DMEM culture medium containing 10% FBS and 1% penicillin/streptomycin.

Sample 2: First, mixing the compound of formula (III) into DMSO to form a mother liquor. Then, diluting the mother liquor to the required concentration for the experiment by means of the DMEM culture medium containing 10% FBS and 1% penicillin/streptomycin.

Sample 3: First, mixing the bakeuchiol derivative with the structure of formula (I) into DMSO to form a mother liquor. Then, diluting the mother liquor to the required concentration for the experiment by means of the DMEM culture medium containing 10% FBS and 1% penicillin/streptomycin.

Sample 4: First, mixing the bakeuchiol derivative with the structure of formula (II) into DMSO to form a mother liquor. Then, diluting the mother liquor to the required concentration for the experiment by means of the DMEM culture medium containing 10% FBS and 1% penicillin/streptomycin.

### Description of Experimental Process

Step 1: Inoculation of HDF (Human Dermal Fibroblasts) cells.

Wherein, inoculating to a 6-well plate at a density of 80 × 10⁴ cells/well, or to be specific, adding to each well 2000 µL of DMEM culture medium containing 10% FBS and 1% penicillin/streptomycin (complete culture medium), and cultivating for 24 hours in a culture box (culture temperature: 37 °C; culture environment: 5% CO₂).

Step 2: Grouping and administering drugs when the cell spreading rate in the 6-well plate reaches 70%~80%.

Wherein, adding complete culture media containing different concentrations of drugs, with 3 sub-wells set up in each group, and placed in a culture box (culture temperature: 37 °C; culture environment: 5% CO₂) after drug administration was completed, cultivating for 24 hours therein.

Step 3: Extracting RNA.

Wherein, (1), pour out the culture medium and use PBS to rinse once. To be specific, firstly, add 1 mL of TansZol^{TM} Up reagent to every 10 cm² of growing cultured cells, place it horizontally for a moment, distribute lysate evenly on the cell surface for cell lysis. Then, use a pipette to blow the cells and make them fall off (wherein, for firmly adhered cultured cells, use a cell scraper to peel them off). Next, transfer the cell lysate to a centrifuge tube and add 0.2 mL of RNA extraction agent (total RNA extraction reagent) and repeatedly blow and suck with a pipette until there is no obvious precipitate in the lysate, then vortex oscillate at room temperature for 5 minutes. (2) Centrifuge the sample between 2 °C and 8 °C for 15 minutes, at which point the sample is divided into three layers: the upper layer (colorless aqueous phase), the middle layer, and the lower layer (pink organic phase). Wherein, RNA is located in the aqueous phase, the volume of the aqueous phase is 50%~60% of the TansZol Up reagent (to avoid inhalation into the middle layer that results in DNA Pollution, a portion of aqueous phase may be retained appropriately). (3) Transfer the colorless aqueous phase to a new centrifuge tube and add an equal volume of anhydrous ethanol (precipitation may occur at this time), and gently mix them well. (4) Add the obtained solution and precipitate into a centrifuge column, centrifuge at room temperature for 30 seconds, and discard the effluent. (5) Add 500 µL of CB9, centrifuge at room temperature for 30 seconds, discard the effluent, and repeat the process once. (6) Add 500 µL of WB9 (before use, check if anhydrous ethanol has been added), centrifuge at room temperature for 30 seconds, discard the effluent, and repeat the process once. (7) Centrifuge at room temperature for 2 minutes to completely remove residual ethanol. (8) Place the centrifuge column in a RNase-freeTube, and add 50-200 µL of Nase-freeWater, let it rest at room temperature for 1 minute in the center of the centrifuge column. (9) Centrifuge at room temperature for 1 minute, wash off the RNA. (10) Use a micro spectrophotometer to conduct concentration measurement of the RNA and store the RNA at -80 °C.

Step 4. Calculating, by 2 ug, the corresponding RNA volume of the RNA sample that has measured concentration, and add the corresponding volume of Rnase free water and reverse transcriptase mix.

Wherein, the total volume of the system is 20 µL. To be specific, the RT-PCR reaction system and the RTPCR reaction program are shown in Table 6:

**Table 6**

| RT-PCR Reaction System | |
|---|---|
| Names | Volumes |
| RNA | 2 ug |
| Rnase Free Water | Up to 20 µL |
| Reverse Transcriptase Mix(5X Evo M-MLV RT Master Mix) | 4 µL |

| RT-PCR Reaction Program | |
|---|---|
| Temperatures | Times |
| 37°C | 40 min |
| 85°C | 5 s |
| 12°C | ∞ |

After the reverse transcription program is completed, dilute 20 µL of the reverse transcription sample to 400 µL, wherein, the final concentration of the cDNA sample is 5 ng/µL (wherein, 3 sub-wells were made for each gene in each sample for testing, and after adding samples, they were put on the machine for QPCR reaction program). To be specific, the QPCR reaction system and the QPCR reaction program are shown in Table 7:

**Table 7**

| QPCR Reaction System (each well volume is 10 µL) | |
|---|---|
| Names | Volumes |
| cDNA Templet | 4 µL |
| 2X QPCR SYBR Green Master Mix | 5 µL |
| ddH₂O | 0.4 µL |
| 10uM Caspase3 Primers (Forward+Reverse) | 0.6 µL |

| QPCR Reaction Program | |
|---|---|
| Temperatures | Times |
| 95°C | 5 min |
| 95°C | 10 s |
| 60°C | 20 s |
| 72°C(scan) | 30 s |
| 40X | |
| Melt(scan) | 15 s |

Wherein, 2-△△Ct is used for analyzing related genes' mRNA expression level, data between groups uses One-way ANOVA statistical analysis, P<0.05 indicates that the difference is significant and has statistical significance.

### Explanation of Experimental Results

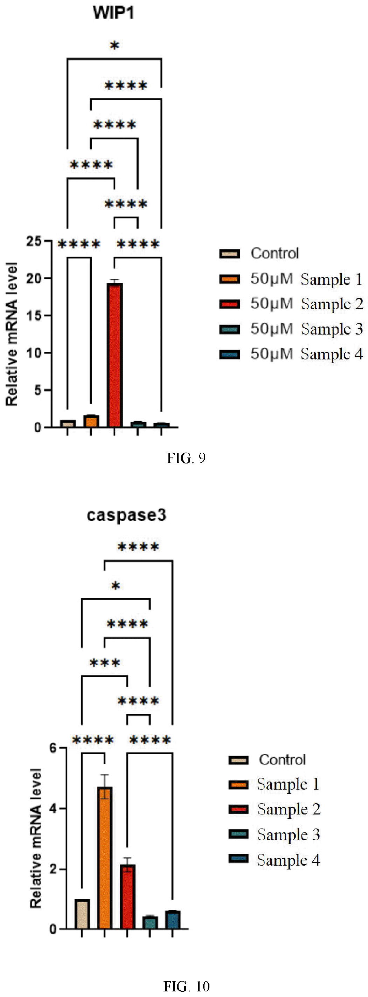

Wherein, the experimental results corresponding to Experiment 4 are shown in Table 8, Figure 8, and Figure 9:

**Table 8**

| Sample Names | WIP1 Relative Expression Level | |
|---|---|---|
| Sample Concentration | 25 µM | 50 µM |
| Control | 1.000 | 1.000 |
| Sample 1 | 1.737 | 1.733 |
| Sample 2 | 9.416 | 19.410 |
| Sample 3 | 0.824 | 0.852 |
| Sample 4 | 0.725 | 0.662 |

Analysis reveals that, firstly, sample 1 improved the WIP1 expression level at both the concentration of 25 µM and 50 µM, increasing by about 70% compared to the control. Secondly, sample 2 at the concentration of 25 µM and 50 µM strongly enhanced the WIP1 expression, increasing by about 800% and 1800% respectively compared to the control. Next, samples 3 and 4 both have the effect of inhibiting the WIP1 when at the concentration of 25 µM and 50 µM, decreasing by about 20~40% compared to the control. In summary, samples 3 and 4, compared to sample 2, have completely different biological activities.

### Experiment 5 (Animal Experiment)

Wherein, Experiment 5 used 25 g ~ 30 g of C57BL/6 male mice (6-8 weeks old, source: Guangdong Experimental Animal Center), which were subjected to a 12-hour light/dark cycle at a temperature of 22 °C and an adaptation period of 4 days before the experiment. To be specific, Experiment 5 used a control (blank control group), a sample 1 (experimental group 1), a sample 2 (experimental group 2), a sample 3 (experimental group 3), and a sample 4 (experimental group 4), with each group containing 5 mice. The specific description of these samples is as follows:
Control: Applying an equal amount of physiological saline solution daily.

Sample 1: Applying a 0.1% aqueous solution of bakeuchiol daily.

Sample 2: Applying a 0.1% aqueous solution of compound of formula (III) mixture daily.

Sample 3: Applying a 0.1% aqueous solution of bakeuchiol derivative with the structure of formula (I) daily.

Sample 4: Applying a 0.1% aqueous solution of bakeuchiol derivative with the structure of formula (II) daily.

Note: On the day before the start of the experiment, except for the blank control group, the mice in the experimental groups had their backs treated with hair removal, with a range of approximately 4 cm × 4 cm. The hair removed site is applied with 200 µL of solution every day. After three days of continuous administration, the experiment ended and the mice were euthanized using the spinal dislocation method, and back skin tissue was collected. The obtained back skin tissue was fixed in a 10% paraformaldehyde solution and stored in a refrigerator at 4 °C. The remaining back skin tissue was frozen in a -80 °C freezer or liquid nitrogen for subsequent indicator analysis.

### Description of Experimental Process

Wherein, Caspase3 content analysis and WIP1 content analysis are included. To be specific:
Caspase3 content analysis includes the following steps:
Step 1: Thoroughly grinding the skin tissue with liquid nitrogen until it is finely ground, and collecting it in an EP tube.

Step 2: Adding RIPA Protein lysate by 10% (mg/mL), crushing the skin tissue using an automatic tissue homogenizer.

Step 3: After crushing, placing the sample on ice and continuing to crack for 30 minutes, during this period, vortex 10 seconds every 10 minutes.

Wherein, the vortex condition is: 4 °C; 12000 × g centrifuge.

Step 4: Collecting the supernatant after completion and using Caspase3 ELISA detection kit for measuring the content of Caspase3 in the skin tissue.

WIP1 content analysis includes the following steps:

Step 1: Thoroughly grinding the skin tissue with liquid nitrogen until it is finely ground, and collecting it in an EP tube.

Step 2: Adding RIPA Protein lysate by 10% (mg/mL), crushing the skin tissue using an automatic tissue homogenizer.

Step 3: After crushing, placing the sample on ice and continuing to crack for 30 minutes, during this period, vortex 10 seconds every 10 minutes.

Wherein, the vortex condition is: 4 °C; 12000 × g centrifuge.

Step 4: Collecting the supernatant after completion and using WIP1 ELISA detection kit for measuring the content of WIP1 in the supernatant.

Wherein, the data between each group uses one-way ANOVA statistical analysis, P < 0.05 indicates that the difference is significant and has statistical significance.

### Explanation of Experimental Results

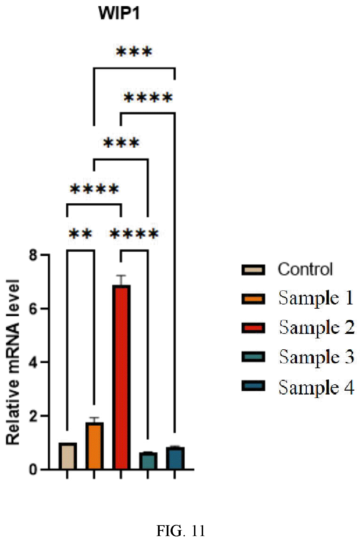

Wherein, the experimental results corresponding to Experiment 5 are shown in Table 9, Table 10, Figure 10, and Figure 11:

**Table 9**

| Groups | Caspase3 Relative Expression Level |
|---|---|
| Control (Blank Control Group) | 1.000 |
| Sample 1 (Experimental Group 1) | 4.723 |
| Sample 2 (Experimental Group 2) | 2.147 |
| Sample 3 (Experimental Group 3) | 0.439 |
| Sample 4 (Experimental Group 4) | 0.625 |

**Table 10**

| Groups | WIP1 Relative Expression Level |
|---|---|
| Control (Blank Control Group) | 1.000 |
| Sample 1 (Experimental Group 1) | 1.772 |
| Sample 2 (Experimental Group 2) | 6.851 |
| Sample 3 (Experimental Group 3) | 0.650 |
| Sample 4 (Experimental Group 4) | 0.863 |

Analysis reveals that, firstly, see the expression level of Caspase3, as compared with the blank control group, the expression level of experimental group 1 increased by nearly 10 times, and the expression level of experimental group 2 also increased by nearly 2 times. However, experimental groups 3 and 4 showed a decrease of over 50%, indicating that they have a significant inhibitory effect. Secondly, see the expression level of Caspase3, as compared with the blank control group, the expression level of experimental group 1 increased by nearly 2 times, experimental group 2 increased by 17 times, while experimental groups 3 and 4 caused the expression level of WIP1 to decrease by 30% and 10%, respectively, indicating that experimental groups 3 and 4 have significant inhibitory effects.

### Description of Application Examples

### Application Example 1

Wherein, Application Example 1 is a lotion prepared based on bakeuchiol derivatives, and the specific formula is shown in Table 11:

**Table 11**

| Raw Material Names | Raw Material Contents (%) |
|---|---|
| Water | To 100 |
| Carbomer | 0.15 |
| Xanthan Gum | 0.15 |
| Isononyl Isononanoate | 10 |
| PEG-100 Stearate | 0.6 |
| Bakeuchiol Derivative with Structure of Formula (I) | 0.1 |
| Triethanolamine | 0.15 |
| Parahydroxyacet-ophenone | 0.5 |
| Hexanediol | 0.5 |
| Butanediol | 3 |

### Application Example 2

Wherein, Application Example 2 is a lotion prepared based on bakeuchiol derivatives, and the specific formula is shown in Table 12:

**Table 12**

| Raw Material Names | Raw Material Contents (%) |
|---|---|
| Water | To 100 |
| Carbomer | 0.15 |
| Xanthan Gum | 0.15 |
| Isononyl Isononanoate | 10 |
| PEG-100 Stearate | 0.6 |
| Bakeuchiol Derivative with Structure of Formula (II) | 0.1 |
| Triethanolamine | 0.15 |
| Parahydroxyacet-ophenone | 0.5 |
| Hexanediol | 0.5 |
| Butanediol | 3 |

### Application Example 3

Wherein, Application Example 3 is a cream prepared based on bakeuchiol derivatives, and the specific formula is shown in Table 13:

**Table 13**

| Raw Material Names | Raw Material Contents (%) |
|---|---|
| Water | To 100 |
| Carbomer | 0.35 |
| Isononyl Isononanoate | 20 |
| PEG-100 Stearate | 1.5 |
| Bakeuchiol Derivative with Structure of Formula (I) | 0.3 |
| Triethanolamine | 0.35 |
| Parahydroxyacet-ophenone | 0.5 |
| Hexanediol | 0.5 |
| Butanediol | 3 |

### Application Example 4

Wherein, Application Example 4 is a cream prepared based on bakeuchiol derivatives, and the specific formula is shown in Table 14:

**Table 14**

| Raw Material Names | Raw Material Contents (%) |
|---|---|
| Water | To 100 |
| Carbomer | 0.35 |
| Isononyl Isononanoate | 20 |
| PEG-100 Stearate | 1.5 |
| Bakeuchiol Derivative with Structure of Formula (II) | 0.3 |
| Triethanolamine | 0.35 |
| Parahydroxyacet-ophenone | 0.5 |
| Hexanediol | 0.5 |
| Butanediol | 3 |

The above description is merely some preferred embodiments of this disclosure and illustrations of the applied technical principles. Those skilled in the art should understand that the scope of the invention involved in the embodiments of this disclosure is not limited to the technical solutions formed by the specific combination of the above technical features, but should cover at the same time, without departing from the above inventive concept, other technical solutions formed by any combination of the above technical features or their equivalent features, for example, technical solutions formed by replacing the above features with technical features of similar functions disclosed in (but not limited to) the embodiments of this disclosure.

All embodiments disclosed herein can be executed separately or in combination with other embodiments, and are considered within the scope of protection claimed by this disclosure.

## Claims

1. A bakeuchiol derivative, comprising:
a bakeuchiol derivative with a structure of formula (I) or a bakeuchiol derivative with a structure of formula (II), wherein the formula (I) is: 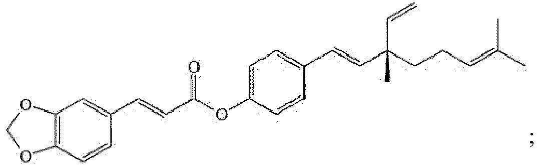
and the formula (II) is:

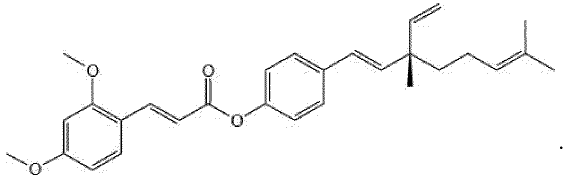

2. A method of preparing a bakeuchiol derivative, comprising:
mixing 3- (benzo [d] [1,3] dioxolan-5-yl) acrylic acid, bakeuchiol, 1-ethyl-(3-dimethylaminopropyl) carbodiimide hydrochloride, 4-dimethylaminopyridine, triethylamine, and tetrahydrofuran, and stirring them evenly at room temperature, so as to obtain a mixture;
performing a first vacuum concentration on the mixture, so as to obtain a first concentrate;
performing extraction on the first concentrate dissolved in water for a target number of times by ethyl acetate, and collecting an organic layer;
performing a second vacuum concentration on a dried organic layer, so as to obtain a second concentrate;
adding petroleum ether to the second concentrate and performing a third vacuum concentration, so as to obtain a first solid;
mixing a mixed solution with the first solid, then beating and filtering to obtain a filter cake, wherein the mixed solution comprises the petroleum ether and the ethyl acetate;
vacuum drying the filter cake to obtain the bakeuchiol derivative, wherein the bakeuchiol derivative is a bakeuchiol derivative with a structure of formula (I) as described in claim 1.

3. A method of preparing a bakeuchiol derivative, comprising:
mixing 2,4-dimethoxycinnamic acid, bakeuchiol, 1-ethyl-(3-dimethylaminopropyl) carbodiimide hydrochloride, 4-dimethylaminopyridine, triethanolamine, and tetrahydrofuran, and stirring them evenly at room temperature, so as to obtain a mixture;
performing a first vacuum concentration on the mixture, so as to obtain a first concentrate;
performing extraction on the first concentrate dissolved in water for a target number of times by ethyl acetate, and collecting an organic layer;
performing a second vacuum concentration on a dried organic layer, so as to obtain a second concentrate;
adding petroleum ether to the second concentrate and performing a third vacuum concentration, so as to obtain a first solid;
mixing a mixed solution with the first solid, then beating and filtering to obtain a filter cake, wherein the mixed solution comprises the petroleum ether and the ethyl acetate;
vacuum drying the filter cake to obtain the bakeuchiol derivative, wherein the bakeuchiol derivative is the bakeuchiol derivative with a structure of formula (II) as described in claim 1.

4. A skincare composition, comprising:
a bakeuchiol derivative, wherein the bakeuchiol derivative is the bakeuchiol derivative with the structure of formula (I) or the bakeuchiol derivative with the structure of formula (II) as described in claim 1; and
a physiologically acceptable excipient or carrier.

5. The skincare composition of claim 4, wherein the bakeuchiol derivative is 0.01% to 5% by weight in a total weight of the skincare composition.

6. The skincare composition of any of claims 4 to 5, wherein the skincare composition further includes one or more selected from: organic solvents, oils, waxes, pigments, fillers, dyes, surfactants, emulsifiers, cosmetic surfactants, organic or inorganic photoprotective agents, polymers, thickeners, preservatives, fragrances, fungicides, actives, retinol, retinol derivatives, flavorings, antioxidants, and rheology modifiers.

7. The skincare composition of any of claims 4 to 5, wherein the skincare composition has a form being one or more selected from: solutions, water agents, lotion, creams, cleaning agents, ointments, perfumes, gel, pastes, powders, sprays, inhalants, and patches.

8. The skincare composition of claim 6, wherein the actives include one or more selected from: small molecules, peptides, proteins, carbohydrates, monosaccharides, oligosaccharides, polysaccharides, nucleoproteins, mucins, synthetic peptides, small molecules linked to proteins, glycoproteins, steroids, nucleic acids, deoxyribonucleic acids, ribonucleic acids, nucleosides, nucleotides, oligonucleotides, antisense oligonucleotides, lipids, hormones, vitamin A, vitamin A derivative, cells.

9. A use of a bakeuchiol derivative, comprising use of the bakeuchiol derivative with a structure of formula (I) or with a structure of formula (II) as described in claim 1 in any of the following:
anti-aging;
inhibiting cell apoptosis;
regulating the cell cycle;
inhibiting deoxyribonucleic acid damage;
inhibiting tumors.

10. A use of a skincare composition, comprising use of the skincare composition as described in any of claims 4 to 8 in any of the following:
anti-aging;
inhibiting cell apoptosis;
regulating the cell cycle;
inhibit deoxyribonucleic acid damage;
inhibiting tumors.
